# EUROPÄISCHE PATENTSCHRIFT

(11) **EP 2 606 660 B1**
(45) Veröffentlichungstag und Bekanntmachung des Hinweises auf die Patenterteilung: **03.05.2017**
(21) Anmeldenummer: 11767219.6
(22) Anmeldetag: 28.09.2011
(51) Int. Cl.: H04R 25/00, A61B 5/12

(54) **VERFAHREN ZUM ANPASSEN EINER HÖRVORRICHTUNG MIT PERZENTIL-ANALYSE**
METHOD FOR FITTING A HEARING DEVICE USING PERCENTILE ANALYSIS
PROCÉDÉ D'ADAPTATION D'UN APPAREIL AUDITIF

(30) Priorität: 30.09.2010 DE 102010041775
(43) Veröffentlichungstag der Anmeldung: 26.06.2013
(73) Patentinhaber: Sivantos Pte. Ltd., Singapore 139959 (SG)
(72) Erfinder: CHALUPPER, Josef, 85307 Paunzhausen (DE)
(74) Vertreter: FDST Patentanwälte
(86) Internationale Anmeldenummer: PCT/EP2011/066863
(87) Internationale Veröffentlichungsnummer: WO 2012/041904

(56) Entgegenhaltungen:
- EP-A2- 0 396 831
- WO-A1-2007/112737
- Parr, Matthias: "Messen von Hörsystem-Funktionselementen", internet , 31. März 2009 (2009-03-31), XP002666358, Gefunden im Internet: URL:http://acousticon.eu/start/index.php?o ption=com_docman&task=doc_download&gid=83 [gefunden am 2011-12-22]
- Harald Bonsel: "Neu Verfahren zur Messung des Dynamikverhaltens von Hörgeräten, Teil 1 - 3", Hörakustik, 30 August 2006 (2006-08-30), XP055186437,
- Peter Kossek ET AL: "Dynamic REM with Percentile analysis", , 30 June 2010 (2010-06-30), XP055186493, Retrieved from the Internet: URL:http://www.otometrics.com/~/media/Down loadLibrary/Otometrics/PDFs/AURICAL/aurica l-dynamic-fitting-real-ear-measurement-7-2 7-0440_00_std.ashx?la=en [retrieved on 2015-04-28]

## Beschreibung

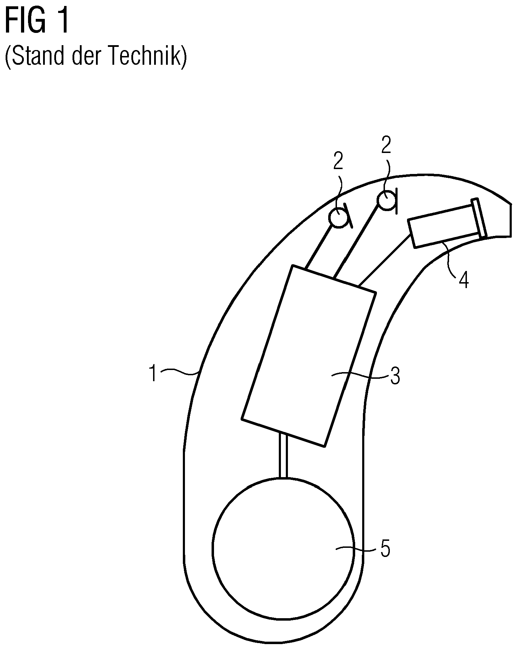

Die vorliegende Erfindung betrifft ein Verfahren zum Anpassen einer Hörvorrichtung. Unter dem Begriff Hörvorrichtung wird hier jedes in oder am Ohr tragbare, schallausgebende Gerät verstanden, insbesondere ein Hörgerät, ein Headset, Kopfhörer und dergleichen.
Hörgeräte sind tragbare Hörvorrichtungen, die zur Versorgung von Schwerhörenden dienen. Um den zahlreichen individuellen Bedürfnissen entgegenzukommen, werden unterschiedliche Bauformen von Hörgeräten wie Hinter-dem-Ohr-Hörgeräte (HdO), Hörgerät mit externem Hörer (RIC: receiver in the canal) und In-dem-Ohr-Hörgeräte (IdO), z.B. auch Concha-Hörgeräte oder Kanal-Hörgeräte (ITE, CIC), bereitgestellt. Die beispielhaft aufgeführten Hörgeräte werden am Außenohr oder im Gehörgang getragen. Darüber hinaus stehen auf dem Markt aber auch Knochenleitungshörhilfen, implantierbare oder vibrotaktile Hörhilfen zur Verfügung. Dabei erfolgt die Stimulation des geschädigten Gehörs entweder mechanisch oder elektrisch.
Hörgeräte besitzen prinzipiell als wesentliche Komponenten einen Eingangswandler, einen Verstärker und einen Ausgangswandler. Der Eingangswandler ist in der Regel ein Schallempfänger, z. B. ein Mikrofon, und/oder ein elektromagnetischer Empfänger, z. B. eine Induktionsspule. Der Ausgangswandler ist meist als elektroakustischer Wandler, z. B. Miniaturlautsprecher, oder als elektromechanischer Wandler, z. B. Knochenleitungshörer, realisiert. Der Verstärker ist üblicherweise in eine Signalverarbeitungseinheit integriert. Dieser prinzipielle Aufbau ist in FIG 1 am Beispiel eines Hinter-dem-Ohr-Hörgeräts dargestellt. In ein Hörgerätegehäuse 1 zum Tragen hinter dem Ohr sind ein oder mehrere Mikrofone 2 zur Aufnahme des Schalls aus der Umgebung eingebaut. Eine Signalverarbeitungseinheit 3, die ebenfalls in das Hörgerätegehäuse 1 integriert ist, verarbeitet die Mikrofonsignale und verstärkt sie. Das Ausgangssignal der Signalverarbeitungseinheit 3 wird an einen Lautsprecher bzw. Hörer 4 übertragen, der ein akustisches Signal ausgibt. Der Schall wird gegebenenfalls über einen Schallschlauch, der mit einer Otoplastik im Gehörgang fixiert ist, zum Trommelfell des Geräteträgers übertragen. Die Energieversorgung des Hörgeräts und insbesondere die der Signalverarbeitungseinheit 3 erfolgt durch eine ebenfalls ins Hörgerätegehäuse 1 integrierte Batterie 5.

Hörschäden können sehr vielfältig ausgeprägt sein. Durch ein Hörgerät muss der jeweilige Hörschaden ausgeglichen werden. Die Hörgeräte besitzen daher zahlreiche Einstellmöglichkeiten. Das jeweilige Hörgerät ist in einem in der Regel aufwendigen Anpassverfahren an den Hörgeräteträger individuell anzupassen.

Um sicherzustellen, dass Nutzsignale wie Sprache oder Musik durch eine Hörgeräteanpassung wieder hörbar gemacht werden, wird eine so genannte Perzentilanalyse verwendet. Bei dieser Perzentilanalyse wird das Übertragungsverhalten des Hörgeräts genauer analysiert. Insbesondere wird das Übertragungsverhalten des Hörgeräts in Perzentilen (Prozenträngen) wiedergegeben. Hierdurch lassen sich Frequenzgänge beispielsweise für laute und leise Pegel ermitteln.

Diese Messmethode mit Perzentilen hat jedoch den Nachteil, dass eine Messung relativ lange dauert, in der Regel über 30 Sekunden. Dies ist insbesondere bei der In-Situ-Anpassung von Hörgeräten nachteilig, da hierbei häufig viele Messungen durchgeführt werden müssen, bis die gewünschte Einstellung erreicht ist. Dies ist für den Hörgeräteträger sehr unkomfortabel.

Nach einer Perzentilanalyse stellt der Akustiker beispielsweise fest, dass bei hohen Frequenzen die Verstärkung bei niedrigen Eingangspegeln erhöht werden sollte. Er wird nun manuell die Kompressionskniepunkte und die Verstärkung in diesem Bereich verändern, jedoch im ersten Schritt in der Regel nicht die gewünschte Änderung optimal erreichen. Dies liegt zum einen daran, dass das Hörgerät ein komplexes, nicht lineares Verhalten besitzt und zum anderen daran, dass das Hörgerät nur begrenzte Einstellmöglichkeiten bietet. Der Akustiker wird also iterativ mehrere Messungen durchführen und möglicherweise abbrechen, bevor die optimale Einstellung erreicht ist, da die Geduld des Hörgeräteträgers nur begrenzt ist.
Bislang musste also diese langwierige Anpassprozedur vom Hörgeräteträger in Kauf genommen werden, oder die Optimierung wurde nicht vollständig beendet. Alternativ dazu wurden bislang auch schnelle, aber nicht alltagsgerechte Messmethoden verwendet, was das Risiko einer schlechten Anpassung erhöht. Aus der Druckschrift EP 0 396 831 A2 ist ein gattungsgemäßes Verfahren zum Anpassen einer Hörvorrichtung bekannt. Es wird ein Frequenzgang der Hörvorrichtung bestimmt, und er wird in Richtung einer Zielverstärkungskennlinie verändert.
Darüber hinaus offenbart der Artikel von Matthias Parr: "Messen von Hörsystem-Funktionselementen", März 2009, URL:http://acousticon.eu/start/index.php? option=com_docman&task=doc_download&gid=83 eine Spracherkennung mit Perzentilanalyse. Die Perzentilanalyse ist für die Signaluntersuchung ein- und ausschaltbar.
Die Aufgabe der vorliegenden Erfindung besteht somit darin, eine qualitativ hochwertige Anpassung in kürzerer Zeit erreichen zu können.
Erfindungsgemäß wird diese Aufgabe gelöst durch ein Verfahren nach Anspruch 1.
In vorteilhafter Weise erfolgt das Anpassen der Hörvorrichtung und insbesondere des Hörgeräts durch ein zweistufiges Verfahren, wobei die Schritte voneinander unabhängig sind. In dem ersten Schritt erfolgt eine absolute Messung der Übertragungseigenschaften der Hörvorrichtung durch die Perzentilanalyse. In einem anschließenden zweiten Schritt wird eine Abweichung zu einer Zielverstärkungskennlinie ermittelt, die Einstellung der Hörvorrichtung verändert und schließlich mit einer einfachen Frequenzgangmessung (ohne Perzentilanalyse) aktuelle Frequenzgang bzw. dessen Änderung ermittelt. Mit der Perzentilanalyse wird also eine sehr exakte Ausgangsbasis geschaffen, während mit der einfachen Frequenzgangmessung die Einstellungsänderungen rasch in ihrer Wirkung überprüft werden können. Insgesamt kann so das Anpassverfahren deutlich beschleunigt werden.

Vor dem Schritt des Änderns erfolgt das gleiche Bestimmen des aktuellen Frequenzgangs wie nach dem Ändern, und daraus wird eine relative Änderung des Frequenzgangs durch das Ändern der Einstellung ermittelt. Dadurch erfolgt die Optimierungsphase mit schnellen Frequenzgangmessungen ohne Perzentilanalyse aufgrund relativer Änderungen.

Am Ende des Anpassverfahrens kann nochmals eine Perzentilanalyse durchgeführt werden. Mit dieser optionalen Analyse kann sichergestellt werden, dass das Anpassziel optimal erreicht wird.

In einer ebenfalls bevorzugten Ausführungsform werden die Schritte des Bestimmens der Abweichung, des Änderns der Einstellung und des Bestimmens des aktuellen Frequenzgangs automatisch durchgeführt. Alternativ können sie natürlich auch manuell oder halbautomatisch durchgeführt werden. Die automatische Durchführung bringt jedoch den Vorteil, dass das gesamte Anpassverfahren weiter beschleunigt werden kann.

In einer speziellen Ausführungsform wird bei dem Bestimmen der Abweichung des Frequenzgangs von einer Zielverstärkungskennlinie über eine Gradientenabschätzung die Summe von Fehlerquadraten minimiert. Auf diese Weise lässt sich die Abweichung effizient und zielgerichtet reduzieren.

Die Einstellung der Hörvorrichtung, die bei der Anpassung geändert wird, kann eine Verstärkung betreffen. Die Hörvorrichtung verändert dann für die Anpassprozedur ihre Verstärkung automatisch, oder sie wird durch den Akustiker manuell verändert.

Die Einstellung der Hörvorrichtung kann aber auch eine Kompression betreffen. Sowohl mit der Änderung der Verstärkung als auch mit der Änderung der Kompression kann erreicht werden, dass der von der Hörvorrichtung gelieferte Schall in den individuellen Hörbereich (unter der Unbehaglichkeitsschwelle und über der Ruheschwelle) des Nutzers der Hörvorrichtung liegt.

Die vorliegende Erfindung wird nun anhand der beigefügten Zeichnungen näher erläutert, in denen zeigen:
- FIG 1: den prinzipiellen Aufbau eines Hörgeräts gemäß dem Stand der Technik;
- FIG 2: ein Ablaufdiagramm des erfindungsgemäßen Verfahrens;
- FIG 3: eine Perzentilanalyse eines Signals und
- FIG 4: das Komprimieren eines Schalls in einen Hörbereich eines Hörers.

Die nachfolgend näher geschilderten Ausführungsbeispiele stellen bevorzugte Ausführungsformen der vorliegenden Erfindung dar.

Die Erfindung basiert auf dem Gedanken, dass die Anzahl der Perzentilmessungen auf eine oder zwei reduziert wird und die zur Realisierung der gewünschten Änderung nötigen Zwischenschritte mit einer schnellen Messung (konventionelle Frequenzgangmessung mit kurzem Rauschsignal oder Sinus-Sweep) durchgeführt werden. Gegebenenfalls können die Zwischenschritte mit der schnellen Messung automatisiert durchgeführt werden.

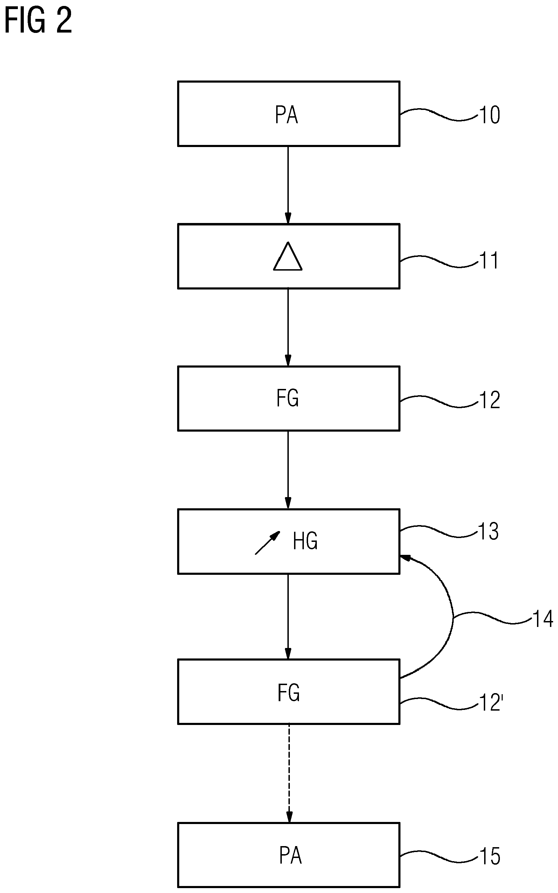

Das Prinzip des erfindungsgemäßen Anpassverfahrens, bei dem das Übertragungsverhalten einer Hörvorrichtung und insbesondere eines Hörgeräts an den Nutzer individuell angepasst werden soll, lässt sich an dem konkreten Beispiel von FIG 2 anschaulich erklären. Es wird zunächst eine Perzentilanalyse 10 der Hörvorrichtung vorgenommen. Dabei wird eine Verstärkungskennlinienschar des Hörgeräts gewonnen. Insbesondere wird dabei der Frequenzgang der Hörvorrichtung für leise und für laute Pegel gewonnen. Die Perzentilanalyse stellt eine sehr genaue, absolute Messung dar. Anschließend erfolgt ein Bestimmen 11 einer Abweichung einer durch die Perzentilanalyse 10 gewonnenen Verstärkungskennlinie von einer Zielverstärkungskennlinie. Beispielsweise kann die Zielverstärkungskennlinie durch die Erfahrung des Akustikers vorgegeben sein, sie kann aber auch aus einem Audiogramm durch eine Anpassformel (z. B. NAL-NL1) gewonnen werden.

Nun wird mit einer ersten Frequenzgangmessung 12 nur auf der Basis eines kurzen Rauschsignals oder eines Sinus-Sweep der Frequenzgang des Hörgeräts gemessen. Diese Messung stellt die Referenz für eine spätere zweite Frequenzgangmessung 12' dar.

In einem anschließenden Schritt erfolgt ein Ändern 13 der Einstellung des Hörgeräts bzw. der Hörvorrichtung. Dieses Einstellen oder Verstellen des Hörgeräts kann automatisch auf der Basis der Abweichung von der Zielverstärkungskennlinie oder manuell (durch Eingabe von vom Akustiker gewünschten Änderungen pro Frequenz und Pegel) erfolgen.

In einem anschließenden Schritt wird die zweite Frequenzgangmessung 12' durchgeführt, wobei die erste und zweite Frequenzgangmessung 12, 12' jeweils wesentlich schneller sind als die Perzentilanalyse 10. Wie bei der ersten Frequenzgangmessung 12 handelt es sich bei der zweiten Frequenzgangmessung 12' um eine gewöhnliche Messung der Übertragungsfunktion des Hörgeräts, bei dem an den Eingang des Hörgeräts beispielsweise ein kurzes Rauschsignal oder ein Sinus-Sweep (Chirp-Signal) angelegt wird. Am Ausgang des Hörgeräts wird dann der entsprechende Ausgangspegel gemessen. Eine derartige Frequenzgangmessung lässt sich in wenigen Sekunden (üblicherweise 2 bis 3 Sekunden) realisieren.

Mit den beiden Frequenzgangmessungen 12, 12' lässt sich eine relative Frequenzgangverschiebung ermitteln, die durch das Ändern 13 der Einstellung hervorgerufen wird. Mit der relativen Verschiebung und der absoluten Messung mit der Perzentilanalyse kann auf den aktuellen Frequenzgang geschlossen werden.

Nach der zweiten Frequenzgangmessung 12' kann, wenn anhand des aktuellen Frequenzgangs festgestellt wird, dass das Anpassungsziel noch nicht erreicht ist, erneut eine Änderung 13 der Hörgeräteeinstellung automatisch oder manuell vorgenommen werden. Da es sich wiederum nur um Änderungen der Einstellungen handelt, kann zur Überprüfung der nun aktuellen Einstellung wieder die schnelle Frequenzgangmessung 12 verwendet werden, weil bei der Messung vor der erneuten Änderung der Hörgeräteeinstellung und danach dieselben Fehler auftreten und sich deshalb wegen des differentiellen Ansatzes aufheben. So erfolgt beispielsweise in dem Hörgerät eine Verfälschung des Signals durch die Störgeräuschbefreiung, wenn die Frequenzgangmessung durch das Rauschsignal am Eingang erfolgt. Wenn jedoch die gleiche Messung mit dem Rauschsignal am Eingang nach einer Veränderung der Einstellung des Hörgeräts erfolgt, kommt es zur gleichen Verfälschung, und die Verfälschungen heben sich bei dem relativen Ansatz auf. Gleiches gilt beispielsweise durch eine Verfälschung wegen einer Rückkopplungsunterdrückung, wenn die Messung mit einem Sinuston (Sinus-Sweep) erfolgt. Des Weiteren können auch Verfälschungen beispielsweise aufgrund des Einschwingverhaltens des Hörgeräts entstehen. Aber auch diese machen sich wegen des differentiellen Ansatzes nicht bemerkbar.

Um die relative Messung nutzen zu können, sind vor und nach einer Änderung 13 der Hörgeräteeinstellung eine Frequenzgangmessung 12 bzw. 12' durchzuführen. Der Pfeil 14 deutet in FIG 2 an, dass das Ändern 13 der Hörgeräteeinstellung und die Frequenzgangmessung 12' beliebig oft wiederholt werden können, um schließlich eine optimale Anpassung zu erreichen. Die jeweils vorhergehende Frequenzgangmessung dient als Referenz für die jeweilige relative Frequenzgangverschiebung.

Vorzugsweise wird also die Sequenz 12', 13 automatisch mehrere Male wiederholt und die Hörgeräteeinstellungen entsprechend den verbleibenden Abweichungen weiter optimiert. Hierbei kann auch ein Optimierungsverfahren verwendet werden, welches über eine Gradientenabschätzung die Summe des Fehlerquadraten minimiert. Abschließend kann optional eine zweite Perzentilmessung 15 zur Verifikation der Einstellung durchgeführt werden.

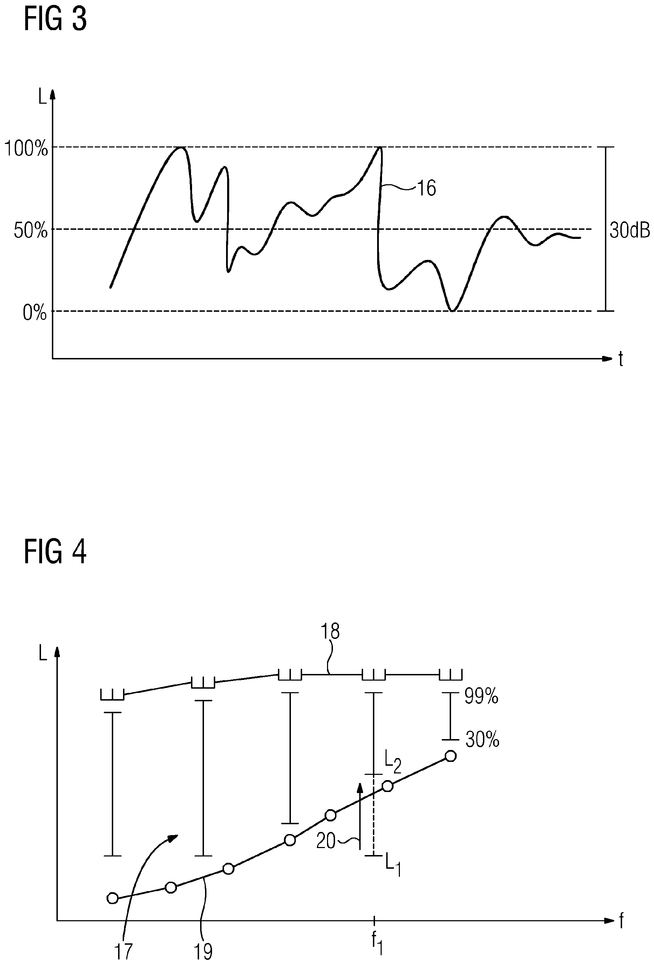

Anhand der FIG 3 und 4 können Details des erfindungsgemäßen Anpassverfahrens anschaulich erklärt werden. FIG 3 zeigt einen Teil einer Perzentilanalyse beispielsweise eines Sprachsignals in einem Frequenzkanal eines Hörgeräts. Das Signal hat beispielsweise in diesem Frequenzkanal einen Dynamikbereich von 30 dB. Die Schnittpunkte des Pegelverlaufs 16 mit der waagrechten 50%-Linie geben an, wie häufig Schall genau mit dem relativen Pegel 50% dargeboten wird (50%-Perzentile). Besonders bedeutsam für die Akustik sind die 99%-Perzentile und die 30%-Perzentile.

Ziel ist es beispielsweise, die 99%-Perzentile und die 30%-Perzentile in den hörbaren Bereich 17 des Hörgeräteträgers zu bringen. Dieser hörbare Bereich 17 befindet sich zwischen der Unbehaglichkeitsschwelle 18 und der Ruhehörschwelle 19. Im vorliegenden Beispiel befinden sich die 30%- und 99%-Perzentilen für alle Frequenzen außer der Frequenz f₁ innerhalb des Hörbereichs 17. Gemäß der Perzentilanalyse befindet sich die 30%-Perzentile bei einem Pegel L₁ unterhalb der Ruhehörschwelle 19. Daher ist in dem Hörgerät für die Frequenz f₁ bzw. den entsprechenden Frequenzkanal die Verstärkung zu erhöhen. Diese Erhöhung ist in FIG 4 mit dem Pfeil 20 symbolisiert. Durch Änderung der Verstärkungseinstellung landet die 30%-Perzentile auf dem Pegel L₂. Diese Verstärkungsänderung kann in einem Schritt oder aber auch in mehreren Einzelschritten erfolgen und sie lässt sich als relative Größe durch einfache Frequenzgangmessungen 12, 12' ermitteln. Letztlich liegt dann die 30%-Perzentile auf einer Zielverstärkungskurve innerhalb des Hörbereichs.

Eine Anpassvorrichtung zur Durchführung des Anpassverfahrens weist beispielsweise eine Analyseeinrichtung auf, mit der die Perzentilanalyse durchgeführt werden kann. Außerdem besitzt sie eine Recheneinrichtung, um eine Abweichung des von der Perzentilanalyse gewonnenen Frequenzgangs von einer Zielverstärkungskennlinie zu bestimmen. Ferner ist eine Stelleinrichtung vorgesehen, um eine Einstellung der Hörvorrichtung bzw. des Hörgeräts insbesondere dahingehend ändern zu können, dass die Abweichung von der Zielverstärkung verringert wird. Schließlich umfasst die Anpassvorrichtung eine von der Analyseeinrichtung unabhängige Messeinrichtung, um den aktuellen Frequenzgang nach der Änderung der Einstellung zu bestimmen, wobei hierzu am Eingang der Hörvorrichtung ein konstantes Rauschsignal oder ein einziger Sinus-Sweep dargeboten und der Ausgangspegel an dem Hörgerät gemessen wird.

In vorteilhafter Weise ermöglicht das erfindungsgemäße Verfahren also eine Anpassung von Hörgeräten mittels qualitativ hochwertiger Perzentilanalyse, wobei das gesamte Anpassverfahren jedoch deutlich durch einfache Frequenzgangmessung(en) beschleunigt ist. Durch dieses mehrstufige Verfahren mit unterschiedlichen Algorithmen lässt sich sehr rasch eine optimale Hörgeräteeinstellung finden.

## Patentansprüche

1. Verfahren zum Anpassen einer Hörvorrichtung durch
- Ausführen einer Perzentilanalyse (10) zum Gewinnen mindestens eines Frequenzgangs der Hörvorrichtung,
- Bestimmen (11) einer Abweichung des Frequenzgangs von einer Zielverstärkungskennlinie,
- Ändern (13) einer Einstellung der Hörvorrichtung, so dass die Abweichung verringert wird,
**gekennzeichnet durch**
- Bestimmen (12, 12') eines aktuellen Frequenzgangs ohne eine Perzentilanalyse nach dem Ändern (13) der Einstellung mittels Darbieten eines konstanten Rauschsignals oder eines einzigen Sinus-Sweep am Eingang der Hörvorrichtung und Pegelmessung am Ausgang,
- wobei vor dem Schritt des Änderns (13) das gleiche Bestimmen (12, 12') des aktuellen Frequenzgangs erfolgt wie nach dem Ändern (13), und daraus eine relative Änderung des Frequenzgangs **durch** das Ändern (13) der Einstellung ermittelt wird.

2. Verfahren nach Anspruch 1, wobei am Ende des Verfahrens nochmals eine Perzentilanalyse (15) durchgeführt wird.

3. Verfahren nach einem der vorhergehenden Ansprüche, wobei die Schritte des Bestimmens (11) der Abweichung, des Änderns (13) der Einstellung und des Bestimmens (12, 12') des aktuellen Frequenzgangs automatisch durchgeführt werden.

4. Verfahren nach einem der vorhergehenden Ansprüche, wobei bei dem Bestimmen (11) der Abweichung über eine Gradientenabschätzung die Summe von Federquadraten minimiert wird.

5. Verfahren nach einem der vorhergehenden Ansprüche, wobei die Einstellung der Hörvorrichtung eine Verstärkung betrifft.

6. Verfahren nach einem der vorhergehenden Ansprüche, wobei die Einstellung der Hörvorrichtung eine Kompression betrifft.

## Claims

1. A method for adjusting a hearing apparatus by
- performing a percentile analysis (10) for obtaining at least one frequency response for the hearing apparatus,
- determining (11) a deviation of the frequency response from a target amplification characteristic curve,
- changing (13) a setting of the hearing apparatus in order to reduce the deviation
chacterized by
- determining (12, 12') an up-to-date frequency response without a percentile analysis after changing (13) the setting by presenting a constant noise signal or a single sinusoidal sweep at the input to the hearing apparatus and measuring a level at the output,
- wherein the same determination (12, 12') of the up-to-date frequency response occurs before the changing (13) step as after changing (13), and a relative change in the frequency response due to changing (13) the setting is determined thereform.

2. The method according to claim 1, which comprises performing a further percentile analysis (15) at the end of the method.

3. The method according to one of the preceding claims, which comprises performing the steps of determining (11) the deviation, changing (13) the setting, and determining (12, 12') the up-to-date frequency response automatically.

4. The method according to one of the preceding claims, wherein the step of determining (11) the deviation includes minimizing the sum of squared errors by way of a gradient estimation.

5. The method according to one of the preceding claims, wherein the setting of the hearing apparatus is an amplification.

6. The method according to one of the preceding claims, wherein the setting of the hearing apparatus is a compression.

## Revendications

1. Procédé pour adapter une prothèse auditive, consistant à :
- effectuer une analyse des percentiles (10) afin d'obtenir au moins une réponse en fréquence de la prothèse auditive,
- déterminer (11) un écart entre la réponse en fréquence et la courbe caractéristique d'amplification cible,
- modifier (13) un réglage de la prothèse auditive de manière à réduire l'écart,
**caractérisé par** le fait de
- déterminer (12, 12') une réponse en fréquence actuelle sans analyse des percentiles après la modification (13) du réglage en présentant un signal de bruit constant ou d'un balayage sinusoïdal unique à l'entrée de la prothèse auditive et en mesurant le niveau à la sortie,
- dans lequel, avant l'étape de modification (13), on effectue la même détermination (12, 12') de la réponse en fréquence actuelle qu'après la modification (13) et on détermine à partir de celle-ci une modification relative de la réponse en fréquence due à la modification (13) du réglage.

2. Procédé selon la revendication 1, dans lequel une analyse des percentiles (15) est de nouveau effectuée à la fin du procédé.

3. Procédé selon l'une quelconque des revendications précédentes, dans lequel les étapes de détermination (11) de l'écart, de modifications (13) du réglage et de détermination (12, 12') de la réponse en fréquence actuelle sont effectuées automatiquement.

4. Procédé selon l'une quelconque des revendications précédentes, dans lequel, lors de la détermination (11) de l'écart, la somme des erreurs quadratiques est minimisée par l'intermédiaire d'une estimation du gradient.

5. Procédé selon l'une quelconque des revendications précédentes, dans lequel le réglage de la prothèse auditive concerne une amplification.

6. Procédé selon l'une quelconque des revendications précédentes, dans lequel le réglage de la prothèse auditive concerne une compression.
